# EUROPEAN PATENT SPECIFICATION

(11) **EP 2 289 522 B1**
(45) Date of publication and mention of the grant of the patent: **25.09.2019**
(21) Application number: 10007406.1
(22) Date of filing: 16.07.2010
(51) Int. Cl.: A61K 31/728, A61P 1/04, A61K 9/08, A61K 9/20

(54) **Mixture of hyaluronic acid for treating and preventing peptic ulcer and duodenal ulcer**
Verwendung von Hyaluronsäure zur Behandlung von Magengeschwüren oder Duodenalulkus
Utilisation d'acide hyaluronique pour le traitement d'ulcères gastro-duodénaux

(30) Priority: 14.08.2009 TW 98127426
(43) Date of publication of application: 02.03.2011
(73) Proprietor: Holy Stone Healthcare Co.,Ltd., Taipei City (TW)
(72) Inventor: Wu, Tsung-Chung, Xindian City, Taipei County (TW)
(74) Representative: Glawe, Delfs, Moll

(56) References cited:
- EP-A1- 1 961 771
- WO-A1-00/01394
- WO-A1-02/09728
- WO-A1-98/10773
- WO-A1-98/48815
- WO-A2-2004/092222
- WO-A2-2008/140499

## Description

### BACKGROUND OF THE INVENTION

### 1. Field of the invention

The present invention provides a mixture of hyaluronic acid for treating and preventing the peptic ulcer and the duodenal ulcer. More particularly, the present invention relates to a mixture comprising at least two or more than two different average hyaluronic acid molecular weights (Mw) and hyaluronic acid with different rheology to gain a hyaluronic acid with the proper adhesion property, functions of tissue scaffold and healing time, in order to treat and to prevent peptic ulcer and the duodenal ulcer and bleeding, thus to achieve the prompt rapidly treatment and to prolong the effect.

The scope of the invention is defined by the claims.

### 2. Description of Related Art

Hyaluronic acid also known as hyaluronan,, hyaluronate and sodium hyaluronate, and generally referred to as HA, which is a natural glycosaminoglycan including the alternative N-acetyl-D-glucosamine and D-glucuronic acid moiety.

The macromolecule of hyaluronic acid in sodium salt form generally is the known composition existing for over fifty years. Referring to Meyer and al (J. Biol Chem. 107,629 (1934)), the hyaluronic acid intrinsically contains the high-viscosity glycosamine alternative with *β* 1-3 glycuronic acid and *β* 1- glycosamine, and the Mw of the high-viscosity glycosamine is between 50,000 Dalton (Da) and few millions Dalton.

We can find the hyaluronic acid in the soft connective tissue in the body of mammals, and the skin, the vitreous humor of the eye, the joint fluid, the umbilical cord and cartilage tissue contains higher volume of the hyaluronic acid.

The hyaluronic acid is widely found in the whole connective tissues, the epithelial cell and the neurotic tissue, and it is the major component of the synovial fluid and is found to increase viscosity of the fluid. The hyaluronic acid is also the major component of the skin and participates in the tissue repair. The hyaluronic acid is rich in extracellular matrix and is helpful for the tissue rheology, movement and the cell proliferation (referring to Delpech, B., Girard, N., Bertrand, P., Courel, M. -N., Chauzy, C., Delpech, A., 1997. Hyaluronan: fundamental principles and applications in cancer. J. Intern. Med. 242,41-48, Rooney, P., Kumar, S., Ponting, J., Wang, M., 1995. The role of hyaluronan in tumour neovascularization. Int. J. Cancer 60, 632-36, Entwistle J, Hall CL, Turley EA Receptors: regulators of signaling to the cytoskeleton. J Cell Biochem 1996; 61: 569-77), and participating the receptor interaction on the surface of some cells; especially the major receptor of CD44. The regulatory function of CD44 is widely accepted as a mark of the activated lymphocyte (referring to Teder P, Vandivier RW, Jiang D, Liang J, Cohn L, Pure E, Henson PM, Noble PW. Resolution of lung inflammation by CD44. Science 2002; 296: 155- 158, Sheehan KM, DeLott LB, Day SM, DeHeer DH, Hyalgan has a dose-dependent differential effect on macrophage proliferation and cell death. J Orthop Res 2003; 21: 744-51).

The hyaluronic acid has the ability of creating and filling due to the organization and modification of the extracellular matrix, and is widely applied in filling the soft tissue for restraining the skin aging caused by age and light, as well as to adjust the obstacle of lipid metabolism on face, and to prevent the recurrent scar or scar formation.

Furthermore, the hyaluronic acid can be applied as the adjuvant agent for the eye operation, or to reduce the pain while movement of the knee and joint of the osteoarthritis patients.

Recently, the hyaluronic acid is applied in clinical treatment in the sodium salt form majorly in the eye, the skin, the surgeon, the artery treatment and the cosmetic fields. The hyaluronic acid with the alkali metal ion, the alkaline earth metal ion (for example the magnesium ion), the aluminum ion, the ammonium ion, and the salt form of the replacement of the ammonium ion can be the carrier for assisting absorption of drug (referring to Belgium Patent 904,547). Among the heavy metal salt of the hyaluronic acid, the silver salt is used as the mycocide and the gold salt is used for treating the rheumatoid arthritis (referring to World Patent WO 87/05517).

The effect of treating the hipsore and the decubitus by the composition (complex) of the hyaluronic acid and the metal ion in the fourth group of periodic table, for example the zinc hyaluronate and the cobalt salt has been proven on the treatment in the Hungary Patent 203,372 to the world.

Bioniche, the Canadian company, disclosed the method and the related structure for using the hyaluronic acid with an effective concentration to treat cystitis in US Patent 5,888,986, wherein the Mw of the hyaluronic acid is more than 200,000 Da. There is only the hyaluronic acid with the certain Mw been applied in the embodiment thereof, for example, to use the hyaluronic acid with the 650 kDa or 1,900 kDa Mw to treat the cystitis; however, the mono molecule of the hyaluronic acid can not be used for both prompt treatment and prolonged effect.

The US patent 2005/0080037 (A1) to Robert Peter Petrella, a Canadian, disclosed the use of hyaluronic acid for treating acute and over sprain and the reaction of soft tissue, wherein the Mw of the hyaluronic acid is only between 90 thousand Da to 120 Da, and a single molecular weight of the hyaluronic acid cannot perform both promptn and immediate healing and prolonged effect.

Richter Gedeon Vegyeszeti Gyar R.T., a Hungarian company, has filed a US Patent 6,656,921 on December 2, 2003 in title of Use of zinc hyaluronate against peptic ulcer to disclose that the zinc hyaluronate with Mw between 500 kDa and 1,200 kDa can be applied to treat peptic ulcer.

Richter Gedeon Vegyeszeti Gyar R.T. also filed a Taiwan Patent application 087106525 on April 28, 1998 entitled Pharmaceutical compositions against peptic ulcer to disclose the purpose of using the zinc composition of the hyaluronic acid (zinc hyaluronate) in the preparation of medical composition with anti peptic ulcer activity and the method of treating and preventing the peptic ulcer. The Mw of the hyaluronic acid thereof is between 790,000 Da and 1.2 million Da. Although this invention has the features of rapidly spreading for treating the peptic ulcer, but the degradation is too quick to keep the drug effect for a longer period of time, therefore, it's inconvenient for the patients in clinical settings.

WO2004/092222 discloses compositions comprising a mixture of low molecular weight hyaluronic acid and high molecular weight hyaluronic acid, and the formulation thereof as an injectable hydrogel.

### SUMMARY OF THE INVENTION

An object of the present invention is to use the biological activity of at least two or more than two average molecular weight of hyaluronic acids in the pharmaceutically acceptable salt to treat peptic ulcer. Due to the low average molecule weight hyaluronic acid (LMWHA) and the high average molecule weight hyaluronic acid (HMWHA) have different adhesive and degradation rate. In this invention, the hyaluronic acid with average Mw lower than 1.5 million Da as LMWHA, and the higher than 1.5 million Da as HMWHA, thus to gain the mixture with LMWHA and HMWHA; the LMWHA can rapidly cover the inflammatory surface to shorten the treatment period of peptic ulcer and the hyaluronic acid, and the HMWHA can prolong the degradation rate in order to maintain a longer effective period, thus to perform both faster and prolonged treatment.

Another object of the present invention is the hyaluronic acid mixture with both LMWHA and HMWHA can conjunctively used with the coagulant, antacid, H2 blocker, the potassium hydrogen ion pump blocker or the mucosa protector as the adjuvant to increase the therapeutic effect.

Another object of the present invention is the hyaluronic acid mixture with both LMWHA and HMWHA can be the major ingredient with proper excipient to formulate a tablet, oral solution or injection solution.

Another object of the present invention is to provide a preferred concentration of the hyaluronic acid mixture with both LMWHA and HMWHA or a pharmaceutically acceptable salt thereof in a range between 0.5 mg/ml to 10 mg/ml, a more preferrable concentration in the solution form in a range from 0.05% to 1% (w/v).

### DETAIL DESCRIPTION OF THE INVENTION

The hyaluronic acid mixture of the present invention used to treat and prevent the peptic ulcer and the duodenal ulcer comprises at least two or more than two average molecular weight of hyaluronic acids with mixed low average molecule weight hyaluronic acid (LMWHA) and the high average molecule weight hyaluronic acid (HMWHA). The different Mw has different rheology, functions of tissue scaffold and degradation in the solution, therefore, the hyaluronic acid mixture can balance the effect and the degradation of HA in order to treat and to prevent peptic ulcer and the duodenal ulcer and bleeding, as well as to have a prompt and rapid effect and a longer treatment effect.

The average Mw lower than 1.5 million Da is categorized as LMWHA, and the range of preferred LMWHA is within 0.5 million to 1.5 million Da; and the average Mw is higher than 1.5 million Da is categorized as HMWHA, and the range of preferred HMWHA is within 1.5 million to 3.5 million Da. The formulation with the LMWHA and the HMWHA can promptly cover the inflammatory portion with the LMWHA to treat the peptic ulcer and the duodenal ulcer, and to sustain the degradation with the HMWHA for extending the effect, thus to achieve prompt treatment and sustained effect.

The general chemical structure of the hyaluronan may be illustrated as follows.

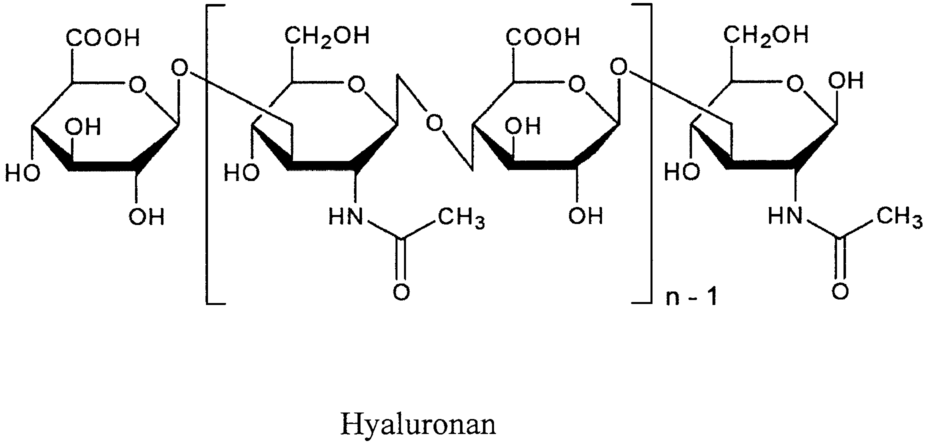

Another preferred embodiment of the present invention is to provide a 1: 1 mixture of the LMWHA and the HMWHA in the salt form of the hyaluronic acid, and adjust the mixture ratio between 20:80 and 80:20 depending on the clinical purpose. The hyaluronic acid mixture with a higher ratio of LMWHA can be more rapidly in the treatment. To the contrary, with a higher ratio of HMWHA can have a better prolonged degradation effect.

Another preferred embodiment of the present invention is to provide a hyaluronic acid mixture including LMWHA and the HMWHA that can be further conjunctively used with a coagulant, antacid, H2 blocker, the potassium hydrogen ion pump blocker or the peptic mucosa protector as the adjuvant to potentiate the therapeutic effect.

Another preferred embodiment of the present invention is to provide a hyaluronic acid mixture including both LMWHA and HMWHA can be a major ingredient with the proper excipient to formulated as oral solid dosage form, the oral solution or suspension or injection solution.

According to the present invention, the oral formulation (for example capsule), the above hyaluronic acid mixture is the active ingredient mixed with excipient (for example starch or carboxy methyl cellulose (CMC)) to form the capsule, the HA mixture will dissolve and disperse in the stomach and form a protective membrane at the peptic ulcer region in order to accelerate healing of the inflammatory region and also to achieve sustained effect.

For oral formulation (for example oral solution), the above hyaluronic acid mixture is the active ingredient mixed with the excipient (for example phosphate buffered saline (PBS solution) or suspension formulation) to form the oral solution, the invented mixture will spread quickly in the stomach and form a protective membrane at the peptic ulcer region in order to accelerate healing of the inflammatory region and also to achieve sustained effect.

For endoscope injection formulation (for example injection solution), the above hyaluronic acid mixture is the active ingredient mixed with the excipient (for example phosphate buffered saline (PBS solution) or suspension formulation) to form the injection solution, the hyaluronic acid mixture can be injected through the endoscope directly into the inflammatory portion to form a protective membrane at the peptic ulcer region in order to accelerate the healing of the inflammatory region and also to achieve sustained effect to shorten the treatment.

The preferred concentration of the hyaluronic acid mixture ratio of the LMWHA and the HMWHA or the pharmaceutically acceptable salt thereof is in a range from 0.5 mg/ml to 10 mg/ml, but the preferred concentration in liquid formulation is in a range from 0.05% to 1% (w/v). The pharmaceutically acceptable salt of the hyaluronic acid mixture is the sodium hyaluronate and the zinc hyaluronate.

According to the above description, at least two or more than two pharmaceutically acceptable salt of the hyaluronic acid may be used to form a hyaluronic acid mixture with different Mw to rapidly forms a coating at the inflammatory surface and prolonged effect by the prolongation of the degradation, and thus achieve a faster treatment and sustained effect to shorten the treatment time.

## Claims

1. A hyaluronic acid mixture for use in treating peptic ulcer and duodenal ulcer comprising at least two hyaluronic acids with a low average molecular weight (LMWHA) and a high average molecule weight (HMWHA) respectively, wherein the average molecular weight (Mw) of said LMWHA is lower than 1.5 million Da, and the average Mw of said HMWHA is higher than 1.5 million Da, and a mixing ratio of said LMWHA and said HMWHA is in a range from 20:80 to 80:20, and wherein said hyaluronic acid mixture includes an excipient to formulate an oral solid dosage form, oral or injection solution; and wherein the administration route is oral or endoscope injection.

2. A hyaluronic acid mixture for use in treating peptic ulcer and duodenal ulcer according to claim 1, wherein said mixing ratio of said LMWHA and HMWHA is 1:1.

3. A hyaluronic acid mixture for use in treating peptic ulcer and duodenal ulcer according to claim 1, wherein said hyaluronic acid mixture is in a concentration range of 0.5 mg/ml to 10 mg/ml.

4. A hyaluronic acid mixture for use according to claim 1, wherein said hyaluronic mixture is formulated as a tablet, an oral solution or an endoscope injectable solution.

5. A hyaluronic acid mixture for use in treating peptic ulcer and duodenal ulcer according to claim 1, wherein said hyaluronic acid mixture is conjunctively used with a coagulant, antacid, H2 blocker, potassium hydrogen ion pump blocker or mucosa protector as an adjuvant to potentiate the drug effect.

6. A hyaluronic acid mixture for use in treating peptic ulcer and duodenal ulcer according to claim 1, wherein said hyaluronic acid can treat and prevent peptic ulcer or duodenal ulcer and bleeding.

## Patentansprüche

1. Hyaluronsäuregemisch zur Verwendung bei der Behandlung von Magengeschwüren und Zwölffingerdarmgeschwüren, umfassend mindestens zwei Hyaluronsäuren mit einem niedrigen Durchschnittsmolekulargewicht (LMWHA) und einem hohen Durchschnittsmolekulargewicht (HMWHA), wobei das Durchschnittsmolekulargewicht (Mw) der LMWHA niedriger als 1,5 Millionen Da ist und das Durchschnittsmolekulargewicht (Mw) der HMWHA höher als 1,5 Millionen Da ist und das Mischungsverhältnis der LMWHA und der HMWHA in einem Bereich von 20:80 bis 80:20 liegt, und wobei das Hyaluronsäuregemisch einen Träger umfasst, um eine orale feste Dosierungsform, eine orale Lösung oder eine Injektionslösung zu formulieren; und wobei der Verabreichungsweg eine orale oder Endoskop-Injektion ist.

2. Hyaluronsäuregemisch zur Verwendung bei der Behandlung von Magengeschwüren und Zwölffingerdarmgeschwüren nach Anspruch 1, wobei das Mischungsverhältnis von LMWHA und HMWHA 1:1 beträgt.

3. Hyaluronsäuregemisch zur Verwendung bei der Behandlung von Magengeschwüren und Zwölffingerdarmgeschwüren nach Anspruch 1, wobei das Hyaluronsäuregemisch in einem Konzentrationsbereich von 0,5 mg/ml bis 10 mg/ml vorliegt.

4. Hyaluronsäuregemisch zur Verwendung nach Anspruch 1, wobei das Hyaluronsäuregemisch als Tablette, eine orale Lösung oder Endoskop-Injektionslösung formuliert ist.

5. Hyaluronsäuregemisch zur Verwendung bei der Behandlung von Magengeschwüren und Zwölffingerdarmgeschwüren nach Anspruch 1, wobei das Hyaluronsäuregemisch zusammen mit einem Koagulans, Antacidum, H2-Blocker, P-Wasserstoffionenpumpenblocker oder Schleimhautschutzmittel als Adjuvans zur Verstärkung der Arzneimittelwirkung verwendet wird.

6. Hyaluronsäuregemisch zur Verwendung bei der Behandlung von Magengeschwüren und Zwölffingerdarmgeschwüren nach Anspruch 1, wobei die Hyaluronsäure Magengeschwüre oder Zwölffingerdarmgeschwüre und Blutungen behandeln und verhindern kann.

## Revendications

1. Mélange d'acides hyaluroniques pour une utilisation dans le traitement de l'ulcère peptique et de l'ulcère duodénal comprenant respectivement au moins deux acides hyaluroniques présentant un poids moléculaire moyen bas (LMWHA) et un poids moléculaire moyen élevé (HMWHA), le poids moléculaire moyen (Mw) dudit LMWHA étant inférieur à 1,5 million Da, et le Mw moyen dudit HMWHA étant supérieur à 1,5 million Da, et un rapport de mélange dudit LMWHA audit HMWHA se situant dans la plage de 20:80 à 80:20, et ledit mélange d'acides hyaluroniques comportant un excipient pour formuler une forme de dosage solide orale, une solution orale ou d'injection ; et le mode d'administration étant une injection orale ou endoscopique.

2. Mélange d'acides hyaluroniques pour une utilisation dans le traitement de l'ulcère peptique et de l'ulcère duodénal selon la revendication 1, ledit rapport de mélange dudit LMWHA au HMWHA étant de 1:1.

3. Mélange d'acides hyaluroniques pour une utilisation dans le traitement de l'ulcère peptique et de l'ulcère duodénal selon la revendication 1, ledit mélange d'acides hyaluroniques se trouvant en une plage de concentration de 0,5 mg/ml à 10 mg/ml.

4. Mélange d'acides hyaluroniques pour une utilisation selon la revendication 1, ledit mélange hyaluronique étant formulé en tant que comprimé, en tant que solution orale ou en tant que solution injectable endoscopique.

5. Mélange d'acides hyaluroniques pour une utilisation dans le traitement de l'ulcère peptique et de l'ulcère duodénal selon la revendication 1, ledit mélange d'acides hyaluroniques étant utilisé conjointement avec un coagulant, un antiacide, un bloqueur des récepteurs H2, un bloqueur de la pompe à ion hydrogène potassium ou un protecteur de muqueuse en tant qu'adjuvant pour renforcer l'effet du médicament.

6. Mélange d'acides hyaluroniques pour une utilisation dans le traitement de l'ulcère peptique et de l'ulcère duodénal selon la revendication 1, ledit acide hyaluronique pouvant traiter et prévenir un ulcère peptique ou un ulcère duodénal et une hémorragie.
